Europäisches Patentamt

European Patent Office

Office européen des brevets

(11) Veröffentlichungsnummer: **0 193 145**

**A2**

(12) # EUROPÄISCHE PATENTANMELDUNG

(21) Anmeldenummer: 86102333.1

(51) Int. Cl.⁴: **A61F 11/04**

(22) Anmeldetag: 22.02.86

(30) Priorität: 26.02.85 DE 3506721

(43) Veröffentlichungstag der Anmeldung:
03.09.86 Patentblatt 86/36

(84) Benannte Vertragsstaaten:
AT BE CH FR GB LI

(71) Anmelder: **Hortmann GmbH**
**6, Robert-Bosch-Strasse**
**D-7449 Neckartenzlingen(DE)**

(72) Erfinder: **Hortmann, Günter**
**Robert-Bosch-Strasse 6**
**D-7449 Neckartenzlingen(DE)**
Erfinder: **Kunick, Klaus**
**Löwentorstrasse 14**
**D-7000 Stuttgart 1(DE)**
Erfinder: **Fenner, Hans**
**Grünlingweg 6**
**D-7000 Stuttgart 70(DE)**

(74) Vertreter: **Möbus, Rudolf, Dipl.-Ing.**
**Hindenburgstrasse 65**
**D-7410 Reutlingen(DE)**

(54) **Übertragungssystem für implantierte Hörprothesen.**

(57) Bei dem Übertragungssystem für implantierte Hörprothesen ist die Sendespule (23) in einem in den Gehörgang (11) einschiebbaren Paßstück (24) angeordnet, und die implantierte Empfangsspule (27) oder der ganze implantierte Empfängerteil (22) ist ringförmig um den Gehörgang (11) des Patienten im Bereich der eingeschobenen Sendespule (23) herumgeführt, wodurch sich eine sichere Kopplungsanordnung von Sendespule und Empfangsspule mit verbessertem Übertragungsfaktor ergibt.

EP 0 193 145 A2

Übertragungssystem für implantierte Hörprothesen

Die Erfindung betrifft ein Übertragungssystem für implantierte Hörprothesen mit mindestens einer Reizelektrode, mit einem akustischen Wandler und angeschlossener Auswerteschaltung, mit einem Hochfrequenzsender, der auf eine Sendespule arbeitet, und mit einem implantierbaren Empfängerteil mit mindestens einer Empfangsspule und mit einem zu der mindestens einen Reizelektrode fürhrenden Empfangskanal.

Zur künstlichen elektrischen Reizung des Innenohres total ertaubter Patienten, deren Hörnerv noch intakt ist, ist es bekannt, eine Mono-oder Multielektrode in die Cochlea einzusetzen, auf welcher mehrere Kontaktkörper verteilt angeordnet sind, die über einen Empfänger durch ein Hochfrequenzsignal von außen erregbar sind und dem Patienten über den Hörnerv einen akustischen Eindruck vermitteln können. Der akustische Wandler liefert die von den aufgenommenen Schallwellen abgeleiteten elektrischen Signale auf eine Auswerteschaltung, deren Aufbau im Rahmen der vorliegenden Erfindung nicht weiter interessiert und deren Aufgabe darin besteht, die Signale so zu formen, aufzuschlüsseln und z. B. auf einzelne Frequenzbandkanäle zu verteilen, daß durch die auf den implantierten Hochfrequenz-Empfänger übertragenen Signale die Reizung des Hörnervs des Patienten möglichst naturgetreu gestaltet wird, so wie sie auch bei der Schallumwandlung im Innenohr erfolgen würde.

Bei der Übertragung der von einem außenliegenden Hochfrequenzsender erzeugten Signale über die Sendespule auf die Empfangsspule eines implantierten Hochfrequenz-Empfängers besteht das Problem, daß eine gute Koppelung zwischen den beiden Spulen erfolgt, damit möglichst wenig Sendeenergie erforderlich wird, die aus einer vom Patienten mitgeführten kleinen Batterie bezogen werden muß. Um hierfür die Sendenspule möglichst dicht an die Empfangsspule heranzubringen, ist es bekannt, die Sendespule an einem Kopfbügel, an einem Kopfband, in einem Brillengestell oder in einem besonderen Ohrbügel so anzuordnen, daß sie dicht über der in dem Bereich hinter der Ohrmuschel implantierten Empfangsspule zu liegen kommt. Eine deckungsgleiche Anordnung von Sendespule und Empfangsspule läßt sich dadurch aber nur zufällig erhalten und wegen der bei der Bewegung des Patienten auftretenden Erschütterungen meist auch nicht lange beibehalten. Es ist zwar versucht worden, eine zentrische Koppelstellung von Sendespule und Empfangsspule durch eine magnetische Halterung der Sendespule zu erreichen, wobei ein besonders starker Magnet aus der Gruppe der seltenen Erden im Zentrum der implantierten Empfangsspule angeordnet wird und ein im Zentrum der außenliegenden Sendespule befindliches Gegenstück anzieht und hält. Hierbei erfolgt jedoch eine dichte Anlage der Sendespule unter Druck gegen die Haut, wodurch leicht Entzündungen im Bereich der implantierten Empfangsspule entstehen können, der ohnehin als Operationsstelle besonders empfindlich ist.

Die bisher bekannte Anordnung der Sende-und Empfangsspule hat außerdem den psychologischen Nachteil, daß die Sendespule oder ihre Halterung sehr auffallend sind und die ohnehin psychisch stark geforderten Gehörlosen in der Gesellschaft kennzeichnen.

Der Erfindung liegt die Aufgabe zugrunde, eine gute elektromagnetische Koppelung zwischen der Sendespule und der Empfangsspule bei einer lagesicheren Anordnung der nichtimplantierten Sendespule zu erzielen, ohne daß hierzu zusätzliche Haltemagnete eingesetzt werden müssen und ohne daß Hautreizungen befürchtet werden müssen.

Die gestellte Aufgabe wird bei einem Übertragungssystem der eingangs genannten Art erfindungsgemäß dadurch gelöst, daß die Sendespule in ein in den Gehörgang des Patienten einschiebbares Paßstück eingearbeitet ist und vom implantierten Empfängerteil mindestens die Empfangsspule im Bereich der eingeschobenen Sendespule ringförmig um den Gehörgang des Patienten gelegt ist. Dabei können entweder die Empfangsspule allein und vom Empfängerteil getrennt oder aber der gesamte Empfängerteil, als Ringkörper ausgebildet, um den Gehörgang gelegt sein.

Der ganze Empfängerteil oder die Empfangsspule allein kann im Zuge des zum Einsetzen der Hörhilfe erforderlichen operativen Eingriffes um den Gehörgang des Patienten gelegt werden. Bei aus dem Empfängerteil ausgesonderter Empfangsspule kann die aus nur wenigen Windungen eines dünnen und medizinisch reinen Silberdrahtes bestehende und eventuell mit einem dünnen Isolationsmantel aus medizinischem Teflon versehene Empfangsspule um den Gehörgang nicht durchtrennt und anschließend wieder zusammengenäht werden muß. Die verwendeten Materialien gewährleisten ein reizloses Einwachsen der Empfängerspule in das Körpergewebe. Auch die Sendespule, die auf einem gemeinsamen Ferritkern angeordnet sein kann, dessen Längsachse zweckmäßig mindestens annähernd senkrecht zur Ebene der Empfangsspule gerichtet wird, läßt sich in dem aus einem hautverträglichen Material gefertigten Paßstück so unterbringen, daß sie von diesem hautverträglichen Material, beispielsweise Silicon, vollständig umschlossen ist. Die genaue Plazierung des Paßstückes im Gehörgang läßt sich entweder durch eine patientenspezifische Ausbildung, also eine genaue Anpassung des Paßstückes an den Gehörgang des einzelnen Patienten, oder aber auf einfachere Weise dadurch gewährleisten, daß das Paßstück über ein in seiner Länge leicht anpaßbares, zugfestes und durch Biegen frei justierbares Isolations-und Schutzrohr mit einem an der Ohrmuschel einhängbaren hakenartigen Ohrbügel verbunden wird, der auch den zweckmäßig als Richtmikrofon ausgebildeten akustischen Wandler trägt. Durch dieses Isolations-und Schutzrohr werden die elektrischen Verbindungsleitungen vom Ohrbügel zur Sendespule geführt. Die Sendespule kann also genau innerhalb der Empfangsspule gehalten werden, so daß sich ein sehr günstiger Übertragungsfaktor ergibt. Es haben sich hierbei Verbesserungen des Übertragungsfaktors bis zu 50 % gegenüber herkömmlichen Spulenkopplungsanordnungen ergeben. Dadurch verringert sich die erforderliche Sendeleistung entsprechend, so daß sich -bei gegebener Batteriekapazität -die Betriebsdauer des Übertragungssystemes beachtlich verlängern oder aber die Batterie verkleinern und damit die ganze Einrichtung kleiner und leichter ausbilden läßt.

Durch die erfindungsgemäße Koppelung von Sendespule und Empfangsspule läßt sich die Einrichtung weitgehend verbergen und läßt am Ohr nicht erkennen, ob es sich um eine Gehörhilfe für Schwerhörige oder für Gehörlose handelt. Bei einem Auskoppeln der Empfangsspule aus dem zu implantierenden Empfänger kann der Empfängerteil an einer beliebigen Stelle auf dem Mastoid verankert werden, was die Operation vereinfacht. Auch üben die Hautdicke über dem Implantat und die Behaarung des Patienten im Bereich des Implantates keinen Einfluß mehr auf die Übertragungsqualität aus. Die Empfangsspule sitzt bei allen Patienten praktisch an der gleichen Stelle, welche für die Sendespule gut zugänglich ist und an welcher die Sendespule einen stabilen Sitz am Körper des Patienten hat, so daß Erschütterungen bei der Bewegung des Patienten, wie sie beispielsweise beim Tanzen auftreten, keine Störung der günstigen Kopplungslage von Sendespule und Empfangsspule bewirken können.

Die durch die optimale Koppelung von Sendespule und Empfangsspule mögliche kleinere Sendeleistung ergibt den weiteren Vorteil, daß eine von der Sendespule ausgehende Störstrahlung nur noch einen vernachlässigbaren Einfluß auf den akustischen Wandler haben kann, so daß das Mikrofon auch bei diesem Übertragungssytem, wie bei Gehörhilfen für Schwerhörige, direkt über dem Ohr angebracht werden kann. Dadurch hat der Patient die Möglichkeit, Schallquellen durch Kopfdrehen gezielt anzupeilen und aus mehreren Schallquellen herauszulösen.

Nachfolgend wird ein Ausführungsbeispiel eines erfindungsgemäß ausgebildeten Mehrfrequenz-Übertragungssystems mit seinen erfindungswesentlichen Teilen anhand der beiliegenden Zeichnung näher erläutert. Im einzelnen zeigen:

Fig. 1 einen schematischen Schnitt durch den Ohrbereich eines Menschen mit Bauteilen des Übertragungssystems;

Fig. 2 eine schematische Darstellung der einzelnen Bauteile des Übertragungssystems in ihrer Zuordnung zueinander.

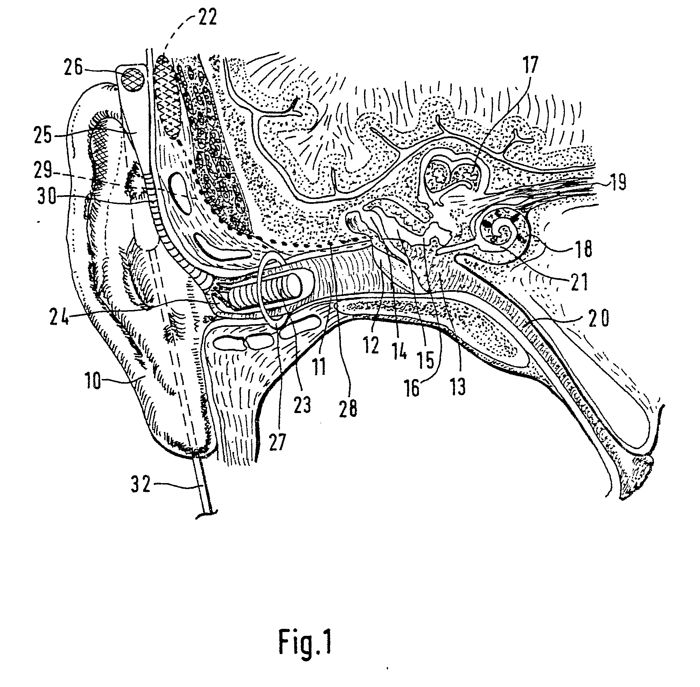

Aus dem medizinischen Schnittbild der Fig. 1 sind die Ohrmuschel 10, der am Trommelfell 12 endende anschließende Gehörgang 11, das Mittelohr mit der Paukenhöhle 13, Hammer 14, Amboß 15, Steigbügel 16, das Labyrinth 17, die Schnecke 18 und der zu ihr führende Hörnerv 19 sowie die Ohrtrompete 20 dargestellt.

Von dem Mehrfrequenz-Übertragungssystem zeigt Fig. 1 eine in die Schnecke eingesetzte Elektrodenplatte 21, einen hinter dem Ohr implantierten Empfängerteil 22, ein die auf einem Ferritkern angeordnete Sendespule 23 aufweisendes, in den Gehörgang eingeschobenes und des Silicon gefertigtes Paßstück 24 sowie einen über die Ohrmuschel gehängten Ohrbügel 25, in welchen als akustischer Wandler ein Richtmikrofon 26 eingebaut ist. Um den Gehörgang 11 herum ist eine ringförmige Emfangsspule 27 implantiert.

Die von dem implantierten Empfängerteil 22 zu einer mehrere Reizelektroden tragenden Elektrodenplatte 21 und zu der Empfangsspule 27 führenden implantierten Verbindungsleitungen sind in Fig. 1 nur schematisch durch strichpunktierte Linien 28 und 29 angedeutet.

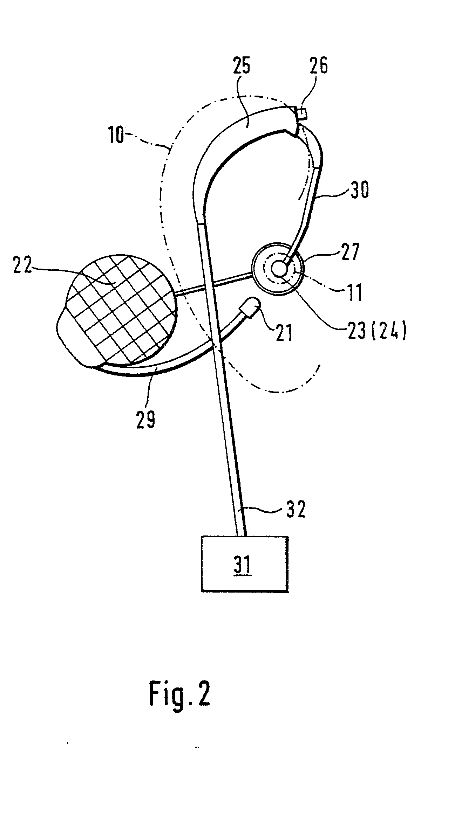

Das die Sendespule 23 tragende Paßstück 24 ist über ein durch Biegen frei justierbares, zugfestes Isolations-und Schutzrohr 30 mit dem Ohrbügel 25 verbunden. In diesem Schutzrohr 30 verläuft die Zuleitung zu der einen senkrecht zur Ebene der Empfangsspule 27 ausgerichteten Ferritkern aufweisenden Sendespule 23. Der Sendeteil 31 (Fig. 2) des Übertragungssystems, mit einer Auswerteschaltung für die vom Mikrofon 26 gelieferten elektrischen Signale und mit einzelnen Hochfrequenzsendern, befindet sich zusammen mit einer Batterie als Betriebsstromquelle in einem von Patienten am Körper, beispielsweise am Gürtel, getragenen Gehäuse. Fig. 1 zeigt nut ein Verbindungskabel 32, das vom Ohrbügel 25 zum Sendeteil 31 führt.

Die Sendespule 23 ist genau innerhalb der implantierten ringförmigen Empfangsspule 27 angeordnet. Die Empfangsspule 27 kann beispielsweise aus nur drei Windungen eines hautverträglich isolierten reinen Silberdrahtes bestehen und einen Durchmesser von ca. 10 mm haben.

Die schematische Darstellung der Fig. 2 zeigt die gesondert angeordneten Einzelteile des Mehrfrequenz-Übertragungssystemes. Von dem Körper des Patienten sind nur die Ohrmuschel 10 und der Gehörgang 11 mit einer strichpunktierten Linie angedeutet. Die Ohrmuschel hält den Ohrbügel 25 mit dem eingebauten Mikrofon 26. Fig 2 zeigt auch die Verbindung des in den Gehörgang 11 eingesetzten, die Sendespule 23 aufweisenden Paßstückes 24 mit dem Ohrbügel 25 über des biegbare Isolations-und Schutzrohr 30 und die konzentrische Anordnung der Empfangsspule 27 zur Sendespule 23. Sie zeigt außerdem den implantierten Sendeteil 22 und die an die Ohrschnecke 18 angesetzte Elektrodenplatte 21 mit ihren mehreren, nicht dargestellten Reizelektroden. Außerdem sind die Verbindungsleitungen 28 und 29, die vom implantierten Empfangsteil 22 zur Empfangsspule 27 oder der Elektrodenplatte 21 führen, sowie das Verbindungskabel 32 zwischen dem Ohrbügel 25 und dem Sendeteil 31 eingezeichnet.

**Ansprüche**

1. Übertragungssystem für implantierte Hörprothesen mit mindestens einer Reizelektrode, mit einem akustischen Wandler und angeschlossener Auswerteschaltung, mit einem Hochfrequenzsender, der auf eine Sendespule arbeitet, und mit einem implantierbaren Empfängerteil mit mindestens einer Empfangsspule und mit einem zu der mindestens einen Reizelektrode führenden Empfangskanal, dadurch gekennzeichnet, daß die Sendespule (23) in ein in den Gehörgang (11) des Patienten einschiebbares Paßstück - (24) eingearbeitet ist und vom implantierten Empfängerteil - (22/27) mindestens die Empfängerspule (27) im Bereich der eingeschobenen Sendespule (23) um den Gehörgang des Patienten gelegt ist.

2. Übertragungssystem nach Anspruch 1, dadurch gekennzeichnet, daß die implantierte Empfangsspule (27) aus wenigen, mit dem implantierten Empfänger (22) verbundenen Windungen reinen Silberdrahtes besteht.

3. Übertragungssystem nach Anspruch 1 oder 2, dadurch gekennzeichnet, daß die Empfangsspule (27) getrennt vom implantierten Empfängerteil (22) angeordnet ist.

4. Übertragungssystem nach Anspruch 1 oder 2, dadurch gekennzeichnet, daß der ganze Empfängerteil (22/27) einschließlich der Empfangsspule (27) als den Gehörgang - (11) umschließender Ringkörper ausgebildet ist.

5. Übertragungssystem nach einem der Ansprüche 1 bis 4, dadurch gekennzeichnet, daß die Sendespule (23) auf einem Ferritkern angeordnet ist, dessen Längsachse mindestens annähernd senkrecht zur Ebene der Empfangsspule gerichtet ist.

6. Übertragungssystem nach einem der Ansprüche 1 bis 5, dadurch gekennzeichnet, daß das die Sendespule (23) aufweisende Paßstück (24) über ein zugfestes, durch Biegen frei justierbares Isolations-und Schutzrohr (30) mit einem den als Richtmikrofon (26) ausgebildeten akustischen Wandler tragenden hakenartigen Ohrbügel (25) zum Einhängen an der Ohrmuschel (10) verbunden ist.

Fig.1

Fig.2